# EUROPÄISCHE PATENTSCHRIFT

(11) **EP 0 510 485 B1**
(45) Veröffentlichungstag und Bekanntmachung des Hinweises auf die Patenterteilung: **04.06.1997**
(21) Anmeldenummer: 92106383.0
(22) Anmeldetag: 14.04.1992
(51) Int. Cl.: G01N 33/00

(54) **Kontinuierlich betriebener Gasanalysator**
Continuous gas analyser
Analysateur de gaz, opéré continuellement

(30) Priorität: 26.04.1991 DE 4113695
(43) Veröffentlichungstag der Anmeldung: 28.10.1992
(73) Patentinhaber: Hartmann & Braun GmbH & Co. KG, 60487 Frankfurt (Main) (DE)
(72) Erfinder: Kahl, Melchior, W-5060 Bergisch Gladbach 2 (DE)
(74) Vertreter: Meissner, Peter E., Dipl.-Ing.

(56) Entgegenhaltungen:
- DD-A- 236 991
- DE-A- 2 932 436
- DATABASE WPI Week 9013 9.Mai 1990 Derwent Publications Ltd., London, GB; AN 90-098210 & SU-A-1 490 132 (DNEPR CHEM TECH) , 24.April 1987

## Beschreibung

Die Erfindung geht aus von einem kontinuierlich betriebenen Gasanalysator mit:
a) einem massenstromabhängigen Detektor,
b) einer Saugvorrichtung am Detektorausgang,
c) einem am Detektorausgang angeschlossenen Unterdruckregelkreis, der den durch die Saugvorrichtung erzeugten Unterdruck p₃ am Detektorausgang gegenüber dem Atmosphärendruck p₀ konstant hält,
d) einer ersten Drossel, die dem Detektor in der Meßgasleitung vorgeschaltet ist,
e) einer zweiten Drossel, die der Reihenschaltung von erster Drossel und Detektor als Beipaß parallel geschaltet ist,
f) einer dritten Drossel, die dem gemeinsamen Gaseintritt vorgeschaltet ist und
g) einem weiteren Unterdruckregelkreis, der an der gemeinsamen Verbindungsstelle der drei Drosseln angeschlossen ist und den dort herrschenden Druck p₂ gegenüber dem Atmosphärendruck p₀ konstant hält.

Ein derartiger, mit Unterdruck betriebener und mit Regelkreisen ausgestatteter Gasanalysator ist in DE 2 932 436 beschrieben. Es hat sich jedoch herausgestellt, daß die Unterdruckregelung verbesserungsbedürftig ist, wenn erhöhte Anforderungen an die Meßgenauigkeit gestellt werden.

Der Erfindung liegt daher die Aufgabe zugrunde, die Regelgenauigkeit bei der Unterdruckregelung im Zusammenhang mit dem vorbeschriebenen kontinuierlich betriebenen Gasanalysator zu verbessern.

Diese Aufgabe wird erfindungsgemäß dadurch gelöst, daß die Unterdruckregelkreise jeweils aus einem piezoelektrischen Druckmeßfühler, einem PI-Regler und einem elektropneumatischen Stellglied bestehen.

Durch die Kombination des piezoelektrischen Druckmeßfühlers mit einem PI-Regler und einem elektropneumatischen Stellglied können die Reproduzierbarkeit, die Temperaturabhängigkeit sowie die Langzeitstabilität der Apparatur um ca. eine Zehnerpotenz verbessert werden.

Im folgenden wird ein Ausführungsbeispiel der Erfindung anhand einer Zeichnung beschrieben. Der prinzipielle Aufbau und die Wirkungsweise der Unterdruckregelung bei einem kontinuierlich betriebenen Gasanalysator wird in DE 2 932 436 ausführlich erläutert. Auf diese Beschreibung wird ausdrücklich Bezug genommen. Dabei werden für die gleichen bzw. analogen Komponenten gleiche Bezugszeichen verwendet. Der wesentliche Unterschied liegt darin, daß die Unterdruckregelung nicht mit konventionellen Differenzdruckreglern arbeitet, sondern mit speziellen elektronischen Regelkreisen 6, 14. Ein solcher Regelkreis besteht jeweils aus einem piezoelektrischen Drucksensor 18a, 18b, einem Meßverstärker 19a, 19b, einem Proportional-/Integralregler 20a, 20b und einem elektropneumatischen Stellglied 21a, 21b. Mit Hilfe des piezoelektrischen Sensors 18a bzw. 18b wird jeweils der Unterdruck strömungsunabhängig mit einer Genauigkeit von ± 0,01 mbar als elektrisches Signal erfaßt. Dieses Signal wird durch den Meßverstärker 19a bzw. 19b verstärkt und dem PI-Regler 20a bzw. 20b zugeführt. Die elektrischen Ausgangssignale der PI-Regler 20a, 20b wirken direkt auf die elektropneumatischen Stellglieder 21a und 21b. Die PI-Regler 20a, 20b führen den Soll-/Istwert-Vergleich durch und erzeugen dementsprechend ein Regelsignal, das die elektropneumatischen Stellglieder 21a, 21b steuert Aufgrund dieser direkten Ansteuerung wird ein hysteresefreies Regelverhalten und eine Regelgenauigkeit von ± 0,01 mbar erreicht. Durch die spezielle Auswahl und Kombination der Regelkreiskomponenten, die besonders gut aufeinander abgestimmt sind, erreicht man eine hohe Langzeitstabilität in einem Temperaturbereich von -10°C bis +70°C. Ein weiterer Vorteil liegt darin, daß über das Regelsignal, d.h. die Abfrage der Stellgrößen, der Meßgasfluß und die Pumpenleistung überwacht werden können, ohne zusätzliche Sensoren oder Durchflußmesser im Meßgasstrom zu installieren. Die beschriebene neue Unterdruckregelung gewährleistet auch einen luftdruckunabhängigen Betrieb des Gasanalysenmeßgerätes, da die beiden Meßfühler als Vergleichsdruck den Luftdruck und damit den gleichen Referenzdruck benutzen.

Die piezoelektrischen Meßfühler und die elektropneumatischen Stellglieder sind handelsübliche Komponenten.

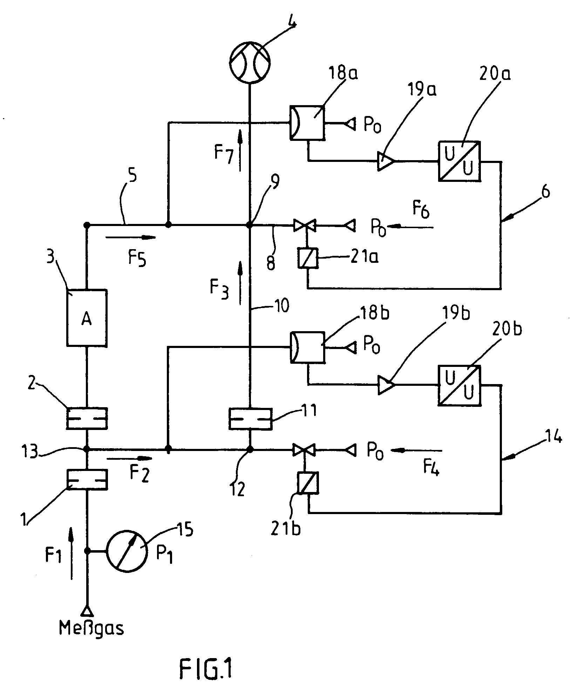

## Patentansprüche

1. Kontinuierlich arbeitender Gasanalysator mit
a) einem massenstromabhängigen Detektor (3),
b) einer Saugvorrichtung (4) am Detektorausgang,
c) einem am Detektorausgang angeschlossenen Unterdruckregelkreis (6), der den durch die Saugvorrichtung (4) erzeugten Unterdruck p₃ am Detektorausgang gegenüber dem Atmosphärendruck p₀ konstant hält,
d) einer ersten Drossel (2), die dem Detektor (3) in der Meßgasleitung vorgeschaltet ist,
e) einer zweiten Drossel (11), die der Reihenschaltung von erster Drossel (2) und Detektor (3) als Beipaß parallel geschaltet ist,
f) einer dritten Drossel (1), die dem gemeinsamen Gaseintritt (12, 13) vorgeschaltet ist und
g) einem weiteren Unterdruckregelkreis (14), der an der gemeinsamen Verbindungsstelle der drei Drosseln (1, 2, 11) angeschlossen ist und den dort herrschenden Druck p₂ gegenüber dem Atmosphärendruck p₀ konstant hält,
dadurch gekennzeichnet, daß die Unterdruckregelkreise (6, 14) jeweils aus einem piezoelektrischen Druckmeßfühler (18a, 18b), einem PI-Regler (20a, 20b) und einem elektropneumatischen Stellglied (21a, 21b) bestehen.

## Claims

1. A continuously operating gas analyser, comprising
a) a detector (3) dependent on mass flow,
b) a suction device (4) at the exit from the detector,
c) a partial-vacuum control circuit (6) connected to the exit from the detector, which circuit keeps the partial vacuum p₃ generated by the suction device (4) at the exit from the detector constant relative to the atmospheric pressure p₀,
d) a first choke point (2), which precedes the detector (3) in the measuring gas line,
e) a second choke point (11), which is connected in parallel to the series connection of first choke point (2) and detector (3) as a bypass,
f) a third choke point (1), which precedes the common gas entry (12, 13) and
g) an additional partial-vacuum control circuit (14) which is connected to the common connection point of the three choke points (1, 2, 11) and keeps the pressure p₂ prevailing therein constant relative to the atmospheric pressure p₀,
characterised in that the partial-vacuum control circuits (6, 14) each consist of a piezoelectric pressure measuring sensor (18a, 18b), a PI controller (20a, 20b) and an electropneumatic actuator (21a, 21b).

## Revendications

1. Analyseur de gaz travaillant de façon continue, comportant :
a) un détecteur (3) dépendant du courant massique,
b) un dispositif d'aspiration (4) à la sortie du détecteur,
c) un circuit de régulation de dépression (6) raccordé à la sortie du détecteur, lequel circuit maintient constante la dépression p₃ engendrée par le dispositif d'aspiration (4) à la sortie du détecteur par rapport à la pression atmosphérique P₀,
d) un premier étranglement (2), qui est monté en amont du détecteur (3) dans le conduit de gaz de mesure,
e) un second étranglement (11), qui est monté parallèlement au montage en série du premier étranglement (2) et du détecteur (3) comme dérivation,
f) un troisième étranglement (1), qui est monté en amont de l'entrée de gaz commune (12,13), et
g) un autre circuit de régulation de dépression (14) qui est raccordé au point de liaison commun des trois étranglements (1,2,11) et maintient constante la pression y régnant p₂ par rapport à la pression atmosphérique p₀,
caractérisé en ce que les circuits de régulation de dépression (6,14) sont constitués, à chaque fois, d'un capteur de mesure de pression piézo-électrique (18a,18b), d'un régulateur proportionnel et par intégration (20a,20b), et d'un organe de réglage électropneumatique (21a,21b).
